# EUROPEAN PATENT APPLICATION

(11) **EP 1 849 799 A1**
(43) Date of publication of application: **31.10.2007**
(21) Application number: 06714349.5
(22) Date of filing: 16.02.2006
(51) Int. Cl.: C07K 14/015, A61K 35/76, A61K 48/00, A61P 43/00, C12N 7/00, C12N 15/09

(54) **MODIFIED VIRUS CAPSID PROTEIN AND THE USE THEREOF**

(30) Priority: 16.02.2005 JP 2005039102
(71) Applicant: THE CIRCLE FOR THE PROMOTION OF SCIENCE AND ENGINE, Meguro-ku Tokyo 1528550 (JP); KONICA MINOLTA HOLDINGS, INC., Chiyoda-ku Tokyo 100-0005 (JP)
(72) Inventor: HANDA, Hiroshi, cho, Midori-ku, Yokohama-shi, Kanagawa, (JP); NAKANISHI, Akira, cho, Midori-ku, Yokohama-shi, Kanagawa, (JP); KANESASHI, Shin-Nosuke, cho, Midori-ku, Yokohama-shi, Kanagawa, (JP); TAKAHASHI, Ryou-u, cho, Midori-ku, Yokohama-shi, Kanagawa, (JP)
(74) Representative: Woods, Geoffrey Corlett
(86) International application number: PCT/JP2006/303209
(87) International publication number: WO 2006/088229

(57) **Abstract**

An altered capsid protein of SV40 that a foreign peptide sandwiched by 1 to several glycine residues each is inserted into at least one of DE-loop and HI-loop of the capsid protein of SV40, and a virus-like particle formed from this protein.

## Description

### Technical Field

The present invention relates to an altered virus capsid protein of SV40 and use thereof. According to the present invention, cell tropism of virus capsid protein, which can be expected to have potential applicability to gene therapy, drug delivery and the like, can be altered by biotechnological procedures. Since the virus capsid protein altered in such way has an ability to form a virus-like particle, particular gene, nucleic acids or pharmaceutically active substances can be encapsulated into the virus-like particle, and thus can be introduced into particular cell, tissue or organ.

### Background Art

Conventional methods of gene transfection and drug delivery by virus vector and virus-like particles had depended on the efficient tropism of virus to the target cell, which is originally possessed by the virus particles. Therefore, the cell species into which each biologically active substance was able to be introduced by suchmethodwere limited, depending on the cell or the organ tropism of the virus particles themselves. To overcome such limitations, development of technology to modify the cell tropism possessed by viruses themselves has long been studied especially with respect to non-enveloped viruses which are mostly composed of only proteins and nucleic acids, generally having stability and long term storage tolerance. As a method of this approach, insertion of a foreign peptide having high cell tropism into a capsid protein of virus can be assumed.

In addition, in mouse polyomavirus (Stubenrauch K, et al. Biochem J. 356:867-873; Shin YC, et al. J Virol., 77:11491-11498) and human papilloma virus (Slupetzky K, et al. J Gen Virol. 82:2799-804; Sadeyen JR, et al. Virology. 309:32-40), a peptide chainwas inserted into each virus capsid protein using similar procedures, and particle formation by some of the capsid proteins having peptide insertion has been observed. However, there has been no report on examining presentation of an inserted peptide chain, particle-forming capability and cell tropism of an altered capsid protein simultaneously, and, the present study is the first time for this kind of study by using SV40:

### Disclosure of Invention

However, when alteration of amino acid sequence of the virus capsid protein was carried out by introducing foreign peptide into amino acid sequence of the virus capsid protein to alter the cell tropism of virus capsid protein, there had been such anxieties that most of the altered virus capsid protein lost particle-forming ability, or that desired characteristics of the altered capsid protein were not exerted because the introduced foreign peptide was not presented on the surface of the virus particle. Therefore, the present invention is directed to controlling specificity and efficiency of the introduction of gene or drug and the like to cells, by finding out an appropriate inserting site for a foreign peptide in the capsid protein of papovavirus which is infectious to primates, particularly of SV40 virus which can be expected to be highly safe to human and have good structural stability, and inserting the foreign peptide into the specified site, to control the cell tropism of the particles formed by the altered capsid protein.

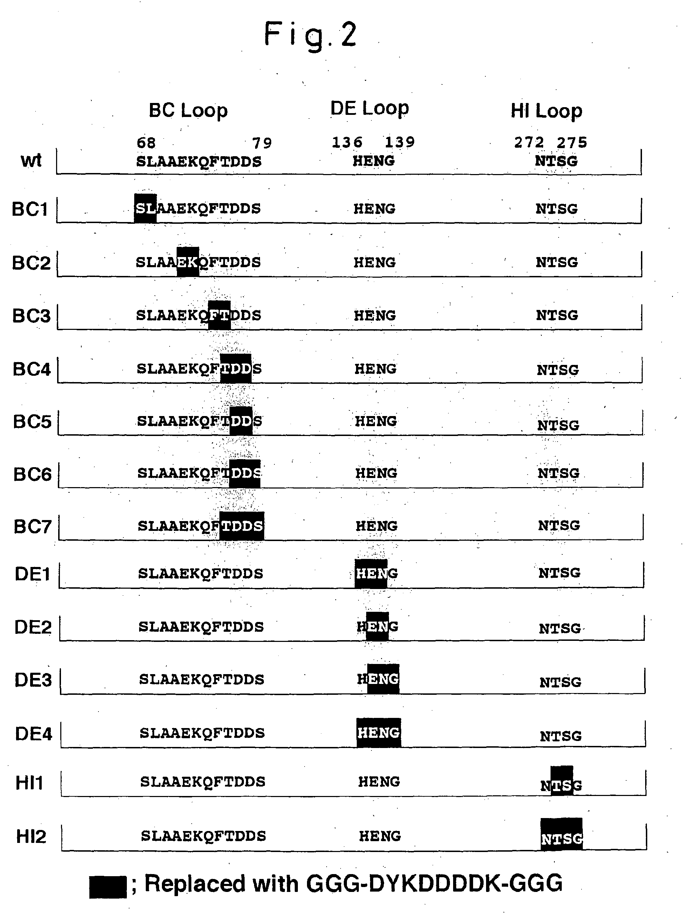

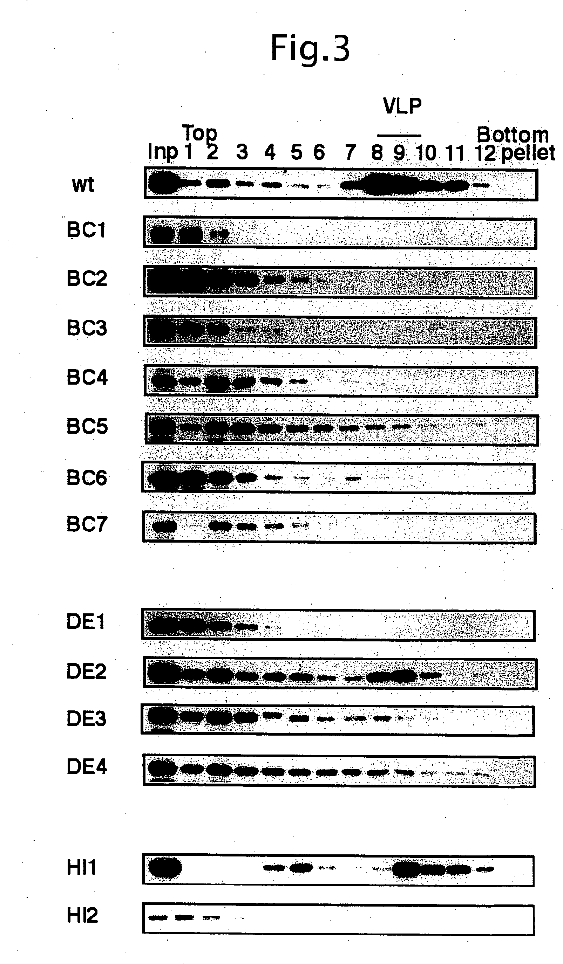

To solve the above-described problems, the present inventors have searched for the inserting site so that the capsid protein can form a particle while the inserted peptide is presented, by inserting a foreign peptide into various regions (refer to Fig: 1 and Fig. 2) of the capsid protein to prepare altered capsid proteins. As the results, from the analyses of about 40 altered proteins (a part of which are shown in Fig. 2), two altered proteins, namely, altered proteins that a foreign peptide sandwiched by 1 to several spacer glycine residues was inserted into DE-loop or HI-loop of the capsid protein of SV40, were found to form particles normally, and the inserted sites where the peptide chain is presented were identified as well (Fig. 2 and Fig. 3). Thus, taking species of virus and range of spacer amino acids, to which this specific alteration technique can be applied, into consideration, the present invention was accomplished.

Thus, the present invention provides an altered capsid protein that a foreign peptide sandwiched by 1 to several spacer amino acid residues is inserted into the amino acid residue constituting the surface of capsid particle of a capsid protein of primate-infective papovavirus. The above-described primate-infective papovavirus includes, for example, SV40, JCB, BKV and the like. The above-described spacer amino acid residue includes, for example, glycine, serine, alanine, and the like.

More specifically, the present invention provides an altered capsid protein of SV40, that a foreign peptide sandwiched by 1 to several glycine residues each is inserted in at least one of DE-loop and HI-loop of the capsid protein of SV40.

The capsid protein of SV40 is preferably SV40 VP1 capsid protein (SEQ ID NO: 13) . The insertion site of the foreign peptide in the DE-loop is preferably positioned between 127^{th} and 146^{th} amino acids counted from the N-terminal of SV40 VP1 capsid protein, and the insertion site in the HI-loop is preferably positioned between 268^{th} and 277^{th} amino acids counted from the N-terminal of SV40 VP1 capsid protein.

Further, another capsid protein, which can be used in the present invention, includes JCV (amino acid sequence is represented by SEQ ID NO: 13), BKV (amino acid sequence is represented by SEQ ID NO: 14), and the like. Expected possible insertion site of the foreign peptide in JCV is in between 119^{th} and 138^{th} amino acids or in between 260^{th} and 269^{th} amino acids counted from the N-terminal. Also, expected possible insertion site of the foreign peptide in BKV is positioned between 127^{th} and 146^{th} amino acids or positioned between 268^{th} and 277^{th} amino acids counted from the N-terminal.

Number of the glycine residue is preferably 1 to 6, for example, 3 to 6 for each side of the foreign peptide.

The foreign peptide includes, for example, Flag sequence (Asp-Tyr-Lys-Asp-Asp-Asp-Asp-Lys) (SEQ ID NO: 1), Myc sequence (Glu-Asn-Lys-Leu-Leu-Ile-Ser-Glu-Glu-Asp-Leu) (SEQ ID NO: 4), HA sequence (Tyr-Pro-Tyr-Asp-Val-Pro-Asp-Tyr-Ala) (SEQ ID NO: 5), Tat-PTD sequence (Arg-Lys-Lys-Arg-Arg-Gln-Arg-Arg-Arg) (SEQ ID NO: 6), polylysine,sequence (3) (Lys-Lys-Lys) (SEQ ID NO:7), polylysine sequence (6) (Lys-Lys-Lys-Lys-Lys-Lys) (SEQ ID NO: 8), polylysine sequence (12) (Lys-Lys-Lys-Lys-Lys-Lys-Lys-Lys-Lys-Lys-Lys-Lys) (SEQ ID NO: 9), NGR sequence (Gln-Gly-Arg) (SEQ ID NO: 10), 6xHis sequence (His-His-His-His-His-His) (SEQ ID NO: 11) or polylysine sequence (8) (Lys-Lys-Lys-Lys-Lys-Lys-Lys-Lys) (SEQ ID NO: 12) or antigen epitope. An example of the antigen epitope includes TAT-PTD sequence of human immune deficiency syndrome (HIV) (Tyr-Gly-Arg-Lys-Lys-Arg-Arg-Gln-Arg-Arg-Arg) (SEQ ID NO: 2) and amino acid sequence 3xRGD comprising integrin recognition sequence (RGD) (Arg-Gly-Asp-Arg-Gly-Asp-Arg-Gly-Asp) (SEQ ID NO: 3).

The present invention also provides a virus-like particle formed from the altered capsid protein of SV40 described above.

The present invention further provides the above-described virus-like particle, in which a biologically active substance is encapsulated. The biologically active substance is, for example, a pharmaceutically active ingredient or a nucleic acid for gene therapy.

Further, the present invention provides a pharmaceutical composition comprising the above-described virus particle.

### Brief Description of the Drawings

Fig. 1 shows the structure of SV40 V1 capsid protein.
Fig. 2 shows the insertion site of Flag sequence in DE-loop, HI-loop and CD loop of SV40 V1 capsid protein. The insertion site is indicated with outline characters on a black background.
Fig. 3 shows whether the virus-like particle is formed or not from the altered type V1 capsid protein having an insertion of Flag sequence into the site indicated in Fig. 2. Bands found at positions 8 to 10 indicate the formation of the virus-like particle.
Fig. 4 shows photomicroscopic picture of virus-like particles formed from wild type V1 capsid protein (wt) , formed from altered type V1 capsid protein having an insertion of Flag sequence into the position of 136^{th} - 139^{th} in DE-loop (DE2) and formed from altered type V1 capsid protein having an insertion of a Flag sequence into the position of 272^{nd} - 275^{th} in HI-loop (HI1).

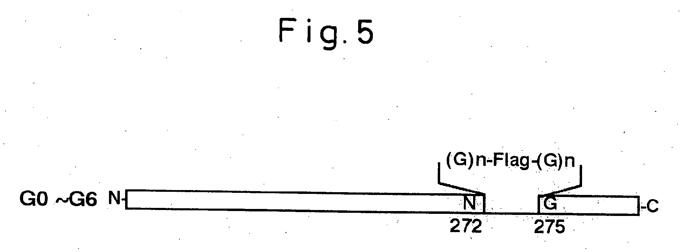

Fig. 5 shows a frame format of the structure of V1 capsid protein having an insertion of Flag sequence sandwiched by n units each of glycine into the position of 272^{nd} 275^{th} in HI-loop.
Fig. 6 shows the relationship between the number of glycine and formation of the virus-like particle. Bands found at positions 8 or 9 indicate the formation of the virus-like particle.
Fig. 7 shows electron microscopic pictures showing the relationship between the number of glycine and formation of the virus-like particle. The particle formation is clearly observed in the range from G3 (3 glycines) to G6 (6 glycines).
Fig. 8 shows the results of immunoprecipitation by anti-Flag antibody of wild type particles (Wt) and particles formed from virus capsid proteins (HI1, DE2) having an insertion of Flag-tag.
Fig. 9 shows the results of detection of proteins in the fractions obtained by sucrose density-gradient centrifugation of a mixture of the particles formed from virus capsid proteins (HI1, DE2) having an insertion of Flag-tag and anti-Flag antibody (upper panel) or anti-His antibody (lower panel).

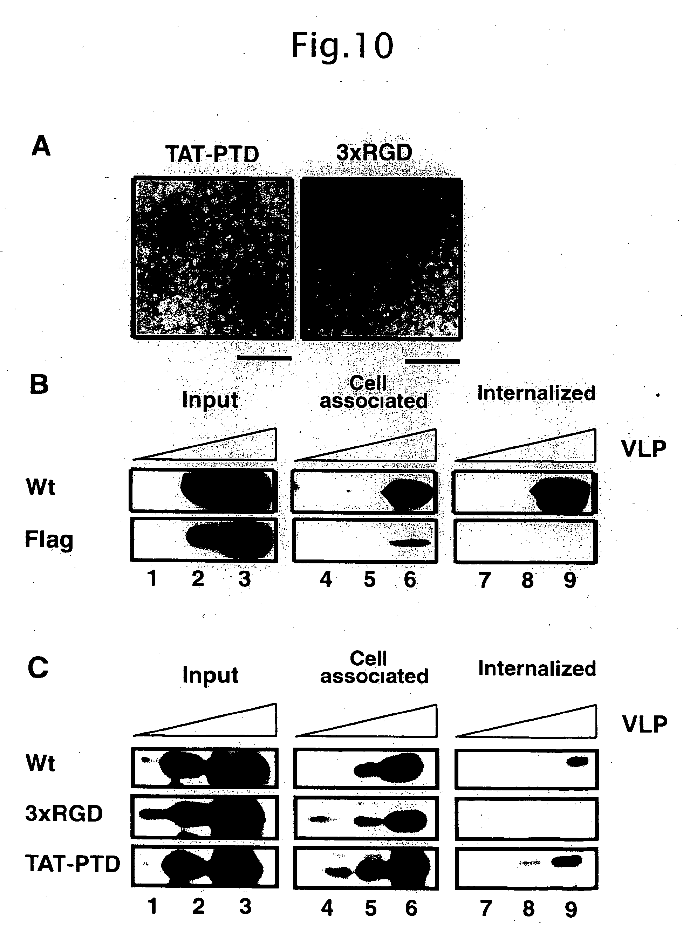

In Fig. 10, A shows the electron microscopic pictures of particles consisted of virus capsid protein presenting TAT-PTD (TAT-PTD-HI1) (left) and 3xRGD (3xRGD-HI1) (right). B shows the results of introduction of Wt particles or Flag-tag inserted particles (Flag-HI1) into CV-1 cells. Introducing capability of the particle into the cell s has been faded away by the insertion of Flag-tag. C shows the results of introduction of Wt particles or the particles having an insertion of 3xRGD or TAT-PTD into CV-1 cells. The particles presenting 3xRGD can adhere to the cells, but internalized particles are not detected when the cells are treated with trypsin. Therefore, observation by a confocal microscope was carried out. Introducing capability of the particles having TAT-PTD was greater than or equivalent to that of wild type particles.
Fig. 11 shows changes of cell tropism among Wt particles and Flag (Flag-DE2) inserted or TAT-PTD (TAT-PTD-DE2) inserted particles. Internalization of VP1 particles into Hela cells is inhibited by the insertion of Flag-Tag into the DE-loop. The particles having an insertion of TAT-PTD into the DE-loop have acquired introducing capability into the cells.
Fig. 12 shows the results of Example 10.
Fig. 13 shows the results of Example 11.

### Best Mode for Carrying Out the Invention

When a pharmaceutically active ingredient or a nucleic acid for gene therapy is introduced into a subject using virus, a point of concern is safety thereof. Many of viruses used as introduction vector are sometimes pathogenic by themselves, and the structural protein of virus sometimes has cytotoxicity. On the other hand, many papovaviruses such as SV40 do not exert pathological change even when infected to the host. Especially, with respect to SV40, in the background of its discovery, the virus has been injected to human in a large scale as a contaminating virus in polio vaccine, and as to the emergency of pathological disorder caused by the injection of SV40, there has been negatively reported.

At the present time, as these viruses are considered to be harmless or to have a very low pathogenesis to human, the capsid protein thereof may be expected to provide a high safety in use for gene transfection into human, drug delivery, or the like, if the cell tropism of capsid protein of the virus can be controlled successfully. Therefore, in the present invention, capsid protein of SV40, preferably VP1 capsid protein of SV40 is utilized.

In the present invention, the insertion site of the foreign peptide is within DE-loop or within HI-loop of the capsid protein of SV40 . In the case of SV40 V1 capsid protein, the DE-loop is locating between 127^{th} and 146^{th} amino acids, and the HI-loop is locating between 268^{th} and 277 ^{th} amino acids counted from the N-terminal of the protein. Preferably, the foreign peptide is inserted in between 137^{th} and 138^{th} amino acid in the DE-loop, or in between 272^{nd} and 275^{th} amino acid in the HI-loop of the SV40 V1 capsid protein.

The foreign peptide to be inserted needs to have 1 to several spacer amino acids, for example, glycine, alanine or serine, and preferably 1 to 9 amino acids, more preferably 1 to 6 amino acids, for example 3 to 6 amino acids are present in each side of the peptide. If the peptide has no such spacer amino acid, formation of virus-like particles becomes difficult.

Preferable foreign peptide is the peptide that can change the cell tropism of a virus-like particle by inserting into the above-described insertion site, and includes Flag sequence (Asp-Tyr-Lys-Asp-Asp-Asp-Asp-Lys) (SEQ ID NO: 1), TAT-PTD sequence as an antigen epitope of human immune deficiency syndrome virus (HIV) (Tyr-Gly-Arg-Lys-Lys-Arg-Arg-Gln-Arg-Arg-Arg) (SEQ ID NO: 2) or amino acid sequence 3xRGD comprising integrin recognition sequence (RGD) (Arg-Gly-Asp-Arg-Gly-Asp-Arg-Gly-Asp) (SEQ ID NO: 3).

The foreign peptide further includes Myc sequence (Glu-Gln-Lys-Leu-Ile-Ser-Glu-Glu-Asp-Leu) (SEQ ID NO: 4), HA sequence (Tyr-Pro-Tyr-Asp-Val-Pro-Asp-Tyr-Ala) (SEQ ID NO: 5), 6xHis sequence (His-His-His-His-His-His) (SEQ ID NO: 11), polylysine sequence (Lys-Lys-Lys-Lys-Lys-Lys-Lys-Lys) (SEQ ID NO: 12), Tat-PTD sequence (Arg-Lys-Lys-Arg-Arg-Gln-Arg-Arg-Arg) (SEQ ID NO: 6), polylysine sequence (3) (Lys-Lys-Lys) (SEQ ID NO: 7), polylysine sequence (6) (Lys-Lys-Lys-Lys-Lys-Lys) (SEQ ID NO: 8), polylysine sequence (12) (Lys-Lys-Lys-Lys-Lys-Lys-Lys-Lys-Lys-Lys-Lys-Lys) (SEQ ID NO: 9), NGR sequence (Gln-Gly-Arg) (SEQ ID NO: 10) and the like.

The present invention also relates to a virus-like particle formed from the above-described altered capsid protein. For the purpose of practical use, a biologically active substance, typically, a pharmaceutically active substance or a nucleic acid for gene therapy is encapsulated into the virus-like particle. The pharmaceutically active substance may be a compound having a low molecular weight, particularly an organic compound having a lowmolecular weight, or a compound having a high molecular weight such as polypeptides, protein or polysaccharides. Examples of such substance include anti-tumor agents such as adriamycin, cyclophosphamide, proteins having anti-tumor activity such as TNFα, Granzyme B, and the like.

In addition, the gene (nucleic acid) for gene therapy is not particularly limited, and includes, for example, human normal genes such as adenosine deamidase gene, p53 gene, and the like; DNA which can express siRNA and suppress specific gene expression by RNA interference; siRNA itself, and the like.

### Examples

The present invention will be explained more specifically by Examples.

### Example 1

### Preparation of altered type VP1 protein

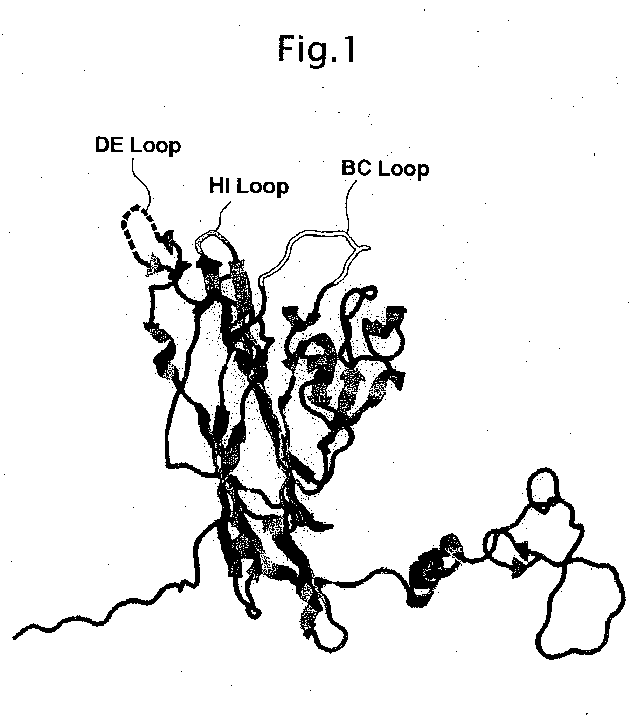

In this Example, focusing on 3 domains, BC-loop, HI-loop and DE-loop among domains presented at the outside of SV40 (see Fig. 1), preparations of altered type SV40-VP1 genes in which foreign epitopes had been inserted were carried out. In the SV40-VP1 protein, a specified amino acid residue on a loop, a part of which was presented at the outside of SV40-VP1 protein, was replaced by the Flag sequence having 3 glycine residues on each end thereof, to prepare the altered type SV40-VP1 gene. The altered gene was inserted into pFastBac1 vector and then transformed into E. coli DH5α.

In the next, Sf9 cells seeded in tissue culture dishes (IWAKI) having a diameter of 10 cm in the population of 1 × 10⁷ cells per dish were infected with recombinant baculoviruses at an m.o.i. (Multiplicity of Infection) of 5 to 10, which can express each of altered type virus protein (VP1) of SV40. The recombinant baculoviruses, which can express each of altered type VP1 proteins, were prepared using Bac-to-Bac Baculovirus Expression System produced by Invitrogen Corp.

At 72 hours after the infection by the recombinant baculoviruses, Sf-9 cells were recovered and washed twice with cooled phosphate buffered saline (PBS). The recovered cells were suspended in 200 µl of ice-cooled buffer for sonication (20mM Tris-HCl (pH 7.9), 1% (w/v) sodium deoxycholate (DOC), 2 mM PMSF, 1 µg/ml chymostatin, aprotinin, leupeptin, antipain, pepstatin), then disrupted by ultrasonication under ice-cooling for 20 seconds, followed by standing on ice bath for 20 seconds. After repeating this procedure 3 times, the disruption mixture was centrifuged under 15,000 x g at 4°C for 10 minutes to recover supernatant solution. Two µl of the recovered VP1 protein was subjected to Western blot analysis using anti-VP1 antibody to determine its expression (Fig. 3).

### Example 2

### Analysis of intracellular formation of virus-like particles of altered type VP1 protein in Sf-9 cells

After determination of the expression, 2 µl of the cell disruption mixture was diluted by 10 times over to 20 µl with 20 mM Tris-HCl (pH 7.9), and then fractionated by 20 to 40% (w/v) sucrose dens ity-gradient centrifugation (at 55, 000 rpm, at 4°C for 1 hour) using Open Top Ultraclear Tube for SW51Ti (Beckman).

Samples of fractions obtained from 20 to 40% (w/v) sucrose density-gradient centrifugation were analyzed forVLP forming capability of the altered VP1 by Western blotting using anti-VP1 antibody. Peak of VP1-VLP formed by wild type VP1 protein (hereinafter, referred to as Wt-VLP) was detected in 8^{th} through 10^{th} fractions (Fig. 3). The similar peak was also observed in the altered type VP1 that the Flag sequence was inserted into DE-loop and HI-loop.

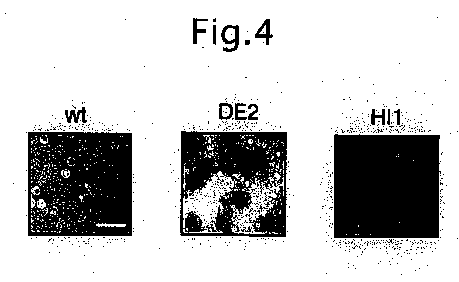

Two hundreds micro litter of the cell disruption mixture was fractionated by 20 to 50% (w/v) cesium chloride density-gradient centrifugation (at 35,000 rpm, at 4°C for 3 hours) using Open Top Ultraclear Tube for SW51Ti (Beckman). After ultracentrifugation, 5 µl each of fractionated samples were transferred and adsorbed on a copper mesh coated with carbon collodion membrane, followed by washing with pure water, then stained using 2 to 6% uranium acetate solution and dried. Observation was carried out using a HITACHI H-7500 Electron Microscope. It was confirmed that the altered type VP1 having Flag sequence insertion in DE-loop or HI-loop formed virus-like particles with almost the same shape and size as that formed from Wt-VLP. The results of analysis on the virus-like particle-forming capability of altered type VP1 were summarized in the following Table 1 (Fig. 4).

**Table 1**

| Loop domain | Particle forming capability | Site replaced by Flag sequence |
|---|---|---|
| BC-loop | ------------ | ----------------------------- |
| DE-loop | VLP was formed | 137^{th} and 138^{th} amino acids from N-terminal |
| HI-loop | VLP was formed | 273^{rd} and 274^{th} amino acids from N-terminal |

### Example 3

### Analysis of functional role of glycine residue in the formation of virus-like particles

In this Example, it was studied what effect the number of glycine residue to be added to each side of Flag sequence inserted into the loop domain gives on the formation of virus-like particles of the altered type VP1.

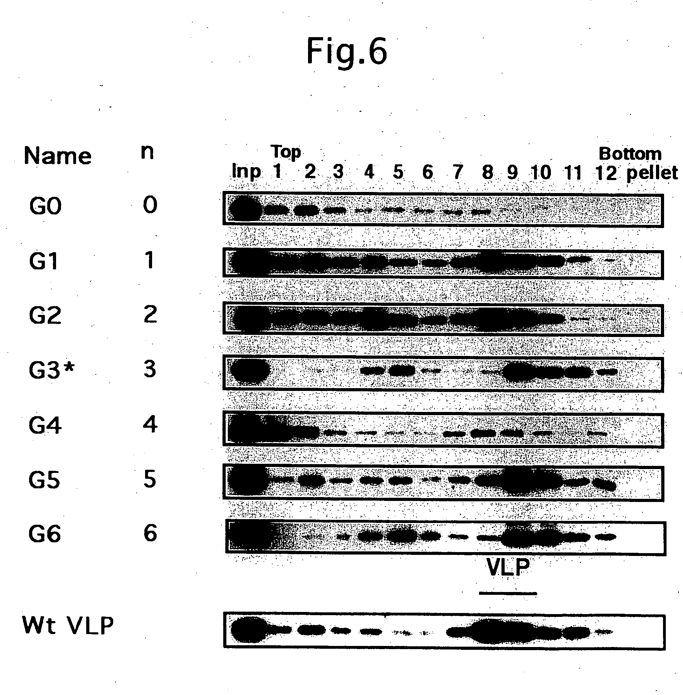

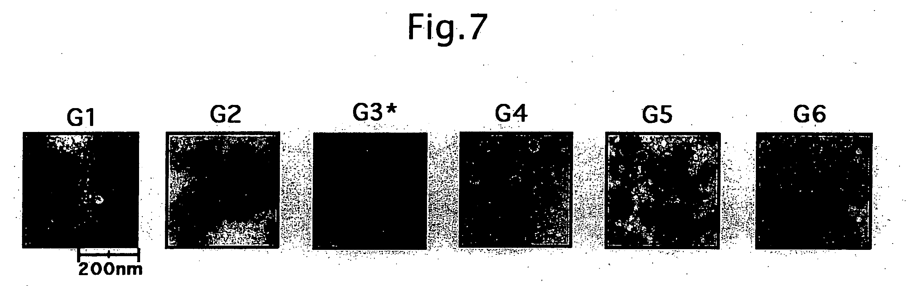

Using HI-loop in the above-described loop domains as target site, altered type VP1 genes inserted with Flag sequence having 0 to 6 glycine residues in each side thereof were prepared according to the same procedures as described in Examples 1 and 2. The results are shown in Fig. 6, fig. 7, and the following Table 2.

**Table 2**

| Number of glycine residue | VLP forming capability |
|---|---|
| 0 | Not detectable |
| 1 | |
| 2 | |
| 3 | VLP was formed |
| 4 | |
| 5 | |
| 6 | |

In addition, from the results of Western blotting analysis of the samples fractionated by sucrose density-gradient centrifugation using anti-VP1 antibody, it was found that efficiency of the formation of virus-like particles of the sample added with 3 glycine residues was better than the sample added with 1 or 2 residues. From the above findings, it was shown that for the formation of virus-like particles by the altered type VP1 inserted with Flag sequence, glysine residue is necessary as a spacer for both sides thereof, and that the number is preferably at least 3 residues.

### Example 4

### Analysis of the function of Flag sequence in the altered type VP1 protein

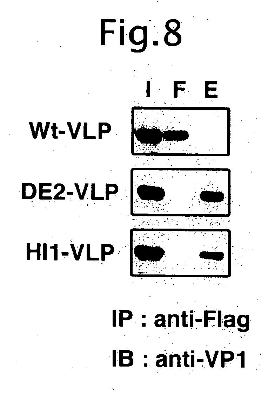

To determine whether the Flag sequence inserted as an epitope into the altered type VP1 works actually as an epitope, immunoprecipitation was carried out using anti-Flag antibody. Twenty µl of altered type virus-like particle (altered type VLP) prepared by the same method as described in the last paragraph of Example 2 was added with phosphate buffered saline (PBS) to give 100 µl, and then agitated gently at 4°C for 1 hour together with 50 µl of anti-Flag M2 Affinity Gel (Sigma). After agitation, the gel was washed twice with 250 µl of phosphate buffered saline (PBS), and then eluted using 1 µg/µl of Flag peptide. The eluate sample was subjected to Western blotting using anti-VPlantibody. By this experiment, it was confirmed that only the altered type VLP having insertion of Flag sequence in the DE-loop and the altered type VLP having insertion of Flag sequence in the HI-loop could bind to anti-Flag antibody (Fig. 8).

### Example 5

### Analysis of the function of Flag sequence in the virus-like particle formed from altered type VP1

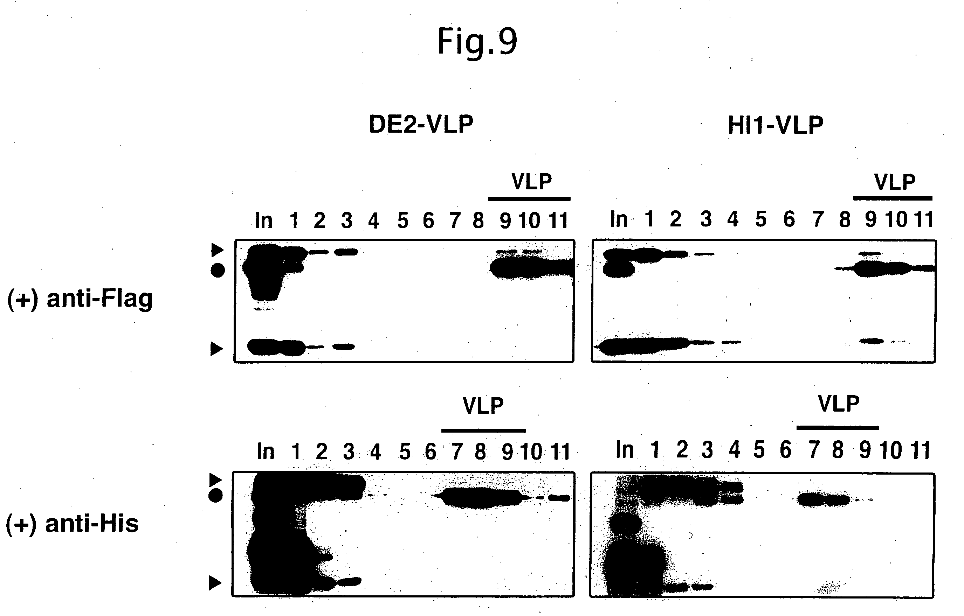

To determine whether the inserted Flag sequence still retain the function as epitope in the altered type VLP even when VLP is formed, the following experiment was carried out. Altered type VLPs of 60 ng each were prepared according to the same procedures as described in Example 1 and Example 2, and each of altered type VLPs was mixed with 9µg of anti-Flag antibody(Sigma)and anti-Hisantibody(Sigma),respectively, and agitated gently at 4°C for 1 hour. After agitation, each mixture was fractionated by 20 to 40% (w/v) sucrose density-gradient centrifugation, followed by Western blotting. When anti-Flag antibody was used, both VP1 and anti-Flag antibody were detected at the peak fractions of fraction number 9 and 10. On the other hand, when anti-His antibody was used, only VP1 was detected at the peak fraction (Fig. 9).

### Example 7

### Large scale purification of altered type VP1 protein

To prepare a large amount of altered type virus-like particles, Sf 9 cells seeded in 10 tissue culture dishes (IWAKI) with a diameter of 15 cm with a population of 3 × 10⁷ cells per dish were infected with each of recombinant baculoviruses, which expresses each altered type virus protein (VP1) of SV40, at an m.o.i. (Multiplicity of Infection) of 5 to 10.

At 72 hours after the recombinant baculovirus infection, Sf-9 cells were recovered and washed twice with cooled phosphate buffered saline (PBS). The recovered cells were suspended in 10 ml of ice-cooled buffer for sonication (20mM Tris-HC1 (pH 7.9), 1% (w/v) sodium deoxycholate (DOC), 2 mM PMSF, 1 µg/ml chymostatin, aprotinin, leupeptin, antipain, pepstatin), then disrupted by ultrasonication under ice-cooling for 10 minutes. After repeating this procedure twice, the cell disruption mixture was centrifuged under 15,000 x g at 4°C for 10 minutes to recover supernatant solution.

In an Open Top Ultraclear Tube for SW41Ti (Beckman), 1.5 ml each of cesium chloride solutions with different densities (50%, 40%, 30%, 20% (w/v)) were overlaid in the order of decreasing density, then 5 ml of the above-described cell disruption mixture was overlaid on the top of the cesium chloride layers. After that, the tube was centrifuged using SW41Ti rotor at 30,000 rpm, at 4°C for 3 hours. The white layer of virus-like particles of SV40 formed in the midpoint of the density gradient was recovered and diluted with 37% (w/v) cesium chloride solution. Thereafter, the diluted solution was transferred to an Open Top Ultraclear Tube for SW55Ti and centrifuged at 50,000 rpm using SW55Ti rotor at 4°C for 20 hours, and the layer of virus-like particles formed again was recovered.

### Example 8

### Analysis of the function of altered type virus-like particle

For the purpose of studying the effects of each inserted motif on the processes of adsorption and internalization of the altered type virus-like particles (Flag virus-like particles, 3xRGD virus-likeparticles, andTAT-PTDvirus-like particles) into the cells, the following experiments were carried out.

HeLa cells (human uterine cervix cancer cells) were seeded in a tissue culture dish (IWAKI) with a diameter of 10 cm in a population of 1 × 10⁶ cells per dish. Also, the wild type virus-like particles and the altered type virus-like particles prepared in Example 7 were diluted with phosphate buffered saline (PBS) to give the concentrations of 4 × 10⁴, 4 × 10⁵, and 4 × 10⁶ particles per cell.

Each of the wild type virus-like particles and the altered type virus-like particles (4 ×10⁴, 4 × 10⁵, and 4 × 10⁶ VLPs/cell) prepared as described above was added with culture medium (DMEM + 10% FBS) to make 1 ml, each, and inoculated on the above described HeLa cells, followed by rocking every 15 minutes at room temperature. After 4 times of rocking, 9 ml each of culture medium was added and incubated at 37°C for 1 hour. Thereafter, the samples were recovered and washed twice with phosphate buffered saline (PBS). The HeLa cells were recovered by two different methods in accordance with the intended use.

### (1) Recovery by scraper

To detect both of the virus-like particles adhered on the surface of HeLa cells and the virus-like particles internalized into the cells, HeLa cells were recovered using a scraper (IWAKI).

### (2) Recovery by trypsin

For the purpose of detecting only internalized virus-like particles, 1 ml of 0.25% Trypsin-EDTA (Gibco) was added to each tissue culture dish with a diameter of 10 cm, and the cells were incubated at 37°C for 5 minutes, then cells detached from the dishes were recovered.

The cells recovered by the above-described method were washed twice with phosphate buffered saline (PBS), and suspended in 200 µl of ice-cooled buffer for sonication, then disrupted by ultrasonication for 60 seconds. The disruption mixture was subjected to Western blotting analysis using anti-VP1 antibody to detect the VP1 proteins both adsorbed on and internalized into the HeLa cells. The results are shown in Table 3.

Also, the results are shown in Fig. 10. In Fig. 10:

A shows the electron microscopic pictures of particles consisted of virus capsid protein in which TAT-PTD or 3xRGD amino acid sequence was inserted into HI-loop thereof. Each particle was almost spherical with a diameter of about 50 nm, and had seemingly no difference from the wild type particle (not shown herein). The black bars at the bottoms indicate 100 nm in length.

Wild type particles and particles consisted of virus capside protein, in which Flag, 3xRGD or TAT-PTD amino acid sequence was inserted, were each infected to CV-1 cells; after standing for 1 hour cells were recovered, then an amount of internalized particles (an amount of VP1) was detected by Western blotting (B and C) . By varying the amount of particles infected as shown by "Input", comparison of the amounts of VP1 detected in the cells was tried. "Cell associated" means that the infected cells were recovered without using proteolytic enzymes such as trypsin, and detection of the particles of both adsorbed on the cell surface and internalized into the cells was tried. "Trypsinized" means that recovery of the infected cells was carried out by trypsin treatment, for the purpose of quantitatively determining only the particles (VP1) remained in the cells by digesting the particles adsorbed on the surface of the cell.

As seen in B and C, Wt particles were able to adsorb on the cells (see Cell associated) and also intrude into the cells (see Trypsinized), while it was observed that the Flag-inserted particles were inhibited at the adsorption step (B). Also, 3xRGD-inserted particles were able to adsorb on the cell similarly to Wt, but internalization thereof was inefficient (C). On the other hand, it was clearly demonstrated that the TAT-PTD-inserted particles were able to intrude into the cells similarly to the Wt particles (C) .

These results indicate that introduction of a peptide chain itself leads to losing the cell tropism of original capsid protein, and also a new cell tropism can be acquired by introducing such a peptide chain as TAT-PTD.

### Example 9

### Alteration of cell tropism by introduction of amino acids

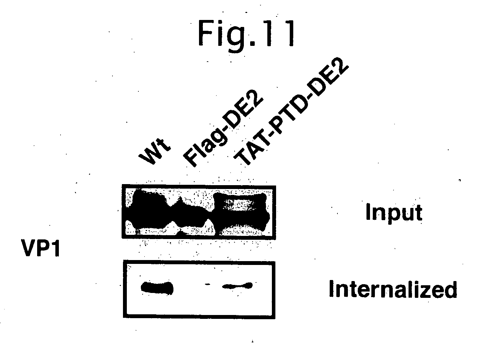

Wild type particles (Wt) or particles consisted of virus protein in which Flag (Flag-DE2) or TAT-PTD (Tat-PTD-DE2) amino acids have been inserted into DE-loop were each infected to HeLa cells, and after 2 hour cells were recovered, then an amount of internalized particles (an amount of VP1) was detected by Western blotting. In the recovery of the cells, the particles adsorbed on the surface of the cells were degraded by trypsin, and only the particles internalized into the cells were detected. As shown by "Input", almost the same amount of particles were used for the introduction into the cells. As shown by "Internalized", the amount of VP1 detected in the cells was almost nothing for Flag-DE2, and slightly lower but comparable for TAT-PTD-DE2 as compared to that for Wt. These results indicate that, as observed similarly when a peptide chain is inserted into HI-loop, introduction of a peptide chain into DE-loop itself leads to losing the cell tropism of original capsid protein, and a new cell tropism can be acquired by introducing such a peptide chain as TAT-PTD (Fig. 11).

### Example 10

### Analysis of the function of altered type virus-like particle (2)

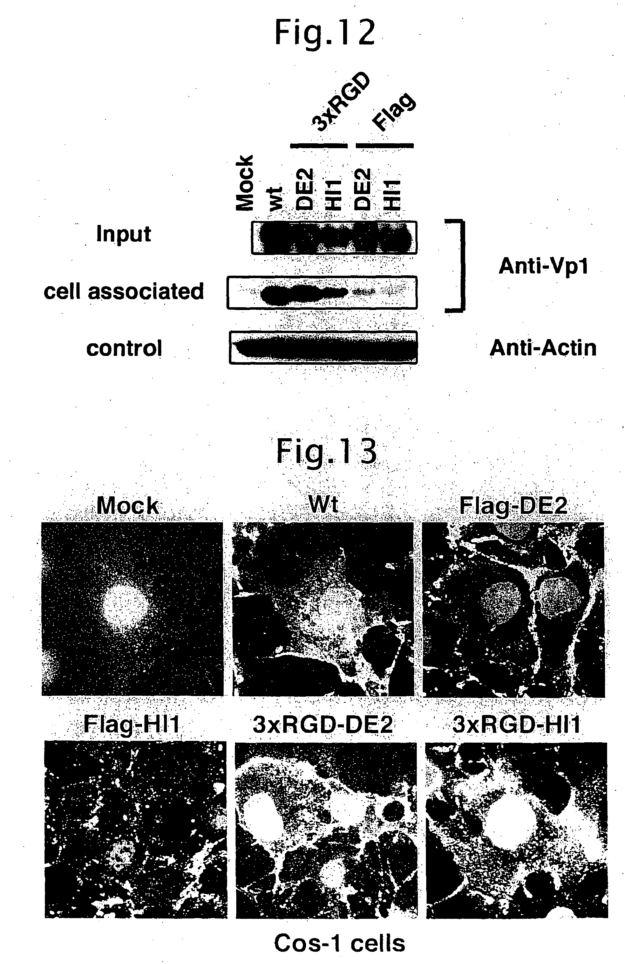

The experiment described in Example 8 was repeated. In this regard, however, instead of the method using trypsin, observation by a confocal microscope was carried out. Flag-HI1 having insertion of Flag sequence in HI-loop, 3xRGD-HI1 having insertion of 3xRGD sequence (RGD-RGD-RGD) in HI-loop, Flag-DE2 having insertion of Flag sequence in DE-loop and 3xRGD-DE2 having insertion of 3xRGD sequence (RGD-RGD-RGD) in DE-loop, and wild type VLP (Wt) were tested. The results of detection using anti-VP1 antibody and observation using a confocal microscope are shown in Fig. 12 and Fig. 13, respectively.

In fig. 13, white spherical body indicates the cell nucleus, and grayish white cloudy area distributed surrounding the nucleus shows assembly of VLPs internalized into cells.

As the results of the above-described experiment, 3xRGD-HI1 and 3xRGD-DE2 having insertion of 3xRGD sequence (RGD-RGD-RGD) in HI-loop or DE-loop were endocytosed by the cells at the same level as observed for wild type VLP. The results are summarized in Table 3.

**Table 3**

| | Adsorption to cell | Internalizatin into cell |
|---|---|---|
| Wild type (Wt) | Yes | Yes |
| Flag sequence (DE2, HI1) | No | No |
| 3xRGD sequence (DE2, HI1) | Yes | Yes/No |
| TAT-PTD sequence (DE2, HI1) | Yes | Yes |

### Conclusion of Examples

TAT-PTD sequence of human immune deficiency syndrome virus (HIV) (Tyr-Gly-Arg-Lys-Lys-Arg-Arg-Gln-Arg-Arg-Arg) (SEQ ID NO: 2) or amino acid sequence 3xRGD comprising integrin recognition sequence (RGD) (Arg-Gly-Asp-Arg-Gly-Asp-Arg-Gly-Asp) (SEQ IDNO:3), or Flag sequence (Asp-Tyr-Lys-Asp-Asp-Asp-Asp-Lys) (SEQ ID NO: 1) and the like were introduced into the surface loops, specifically between 136^{th} and 139^{th} or between 272^{nd} and 275^{th} of VP1 amino acid sequence of SV40 capsid protein, and then particles were formed with these altered type VP1s. The altered type VP1s were able to form particles regardless of the amino acid sequence introduced.

In addition, the inserted amino acid sequences were able to be recognized by antibody without making altered type VP1 denatured, and therefore, those sequences were presented on the surface of the particles. Using such particles, introduction into CV-1 cells or HeLa cells was tested. As the results, the Flag-inserted altered type particles were mostly unable to adsorb to the cells. On the other hand, the particles inserted with 3xRGD were able to adsorb to the cells, but the internalization efficiency thereof was lower compared to that of the wild type. However, the particles inserted with TAT-PTD sequence were able to adsorb to the cells and internalized, and efficiency thereof was slightly higher than that of the particles comprising wild type VP1. Namely, it is indicated that the cell tropism originally possessed by virus capsid protein is inactivated by insertion of a peptide chain, but a new cell tropism is provided by the function of inserted peptide chain.

### Industrial Applicability

By the insertion of a foreign peptide chain into the right position of virus capsid protein, efficient presentation of the peptide chain could be achieved without inhibiting particle formation of the capsid protein. By this insertion of a peptide chain, the cell tropism originally possessed by the virus capsid protein was inhibited, and the cell tropism of the particle could be controlled with the inserted peptide chain.

## Claims

1. An altered capsid protein, wherein a foreign peptide sandwiched with 1 to several spacer amino acid residues is inserted to amino acid residue constituting a surface of a capsid particle of a capsid protein of primate-infective papovavirus.

2. The altered capsid protein according to claim 1, wherein the primate-infective papovavirus is SV40, JCV or BKV.

3. The altered capsid protein according to claims 1 or 2, wherein the spacer amino acid residue is glycine, serine or alanine.

4. The altered capsid protein of SV40, wherein a foreign peptide sandwiched with 1 to several glycine residues is inserted into at least either of DE-loop and HI-loop of a capsid protein of SV40.

5. The altered capsid protein of SV40 according to claim 4, wherein the capsid protein of SV40 is SV40 VP1 capsid protein.

6. The altered capsid protein of SV40 according to claim 5, wherein the insertion site of foreign peptide in the DE-loop is positioned between 127^{th} amino acid and 146^{th} amino acid counted from the N-terminal of SV40 VP1 capsid protein, and the insertion site of foreign peptide in the HI-loop is positioned between 268^{th} amino acid and 277^{th} amino acid counted from the N-terminal of SV40 VP1 capsid protein.

7. The altered capsid protein of SV40 according to any one of claims 4 to 6, wherein the number of glycine residues is 1 to 6 for each side of the foreign peptide.

8. The altered capsid protein of SV40 according to claim 7, wherein the number of glycine residue is 3 to 6 for each side of the foreign peptide.

9. An altered capsid protein of SV40 according to any one of claims 4 to 8, wherein the foreign peptide is Flag sequence (Asp-Tyr-Lys-Asp-Asp-Asp-Asp-Lys) (SEQ ID NO: 1), Myc sequence (Glu-Asn-Lys-Leu-Leu-Ile-Ser-Glu-Glu-Asp-Leu) (SEQ ID NO: 4), HA sequence (Tyr-Pro-Tyr-Asp-Val-Pro-Asp-Tyr-Ala) (SEQ ID NO: 5), Tat-PTD sequence (Arg-Lys-Lys-Arg-Arg-Gln-Arg-Arg-Arg) (SEQ ID NO: 6), polylysine sequence (3) (Lys-Lys-Lys) (SEQ ID NO:7), polylysine sequence (6) (Lys-Lys-Lys-Lys-Lys-Lys) (SEQ ID NO: 8), polylysine sequence (12) (Lys-Lys-Lys-Lys-Lys-Lys-Lys-Lys-Lys-Lys-Lys-Lys) (SEQ ID NO: 9), NGR sequence (Gln-Gly-Arg) (SEQ ID NO: 10), 6xHis sequence (His-His-His-His-His-His) (SEQ ID NO: 11) or polylysine sequence (8) (Lys-Lys-Lys-Lys-Lys-Lys-Lys-Lys) (SEQ ID NO: 12).

10. The altered capsid protein of SV40 according to any one of claims 4 to 8, wherein the foreign peptide comprises antigenic epitope.

11. The altered capsid protein of SV40 according to any one of claims 4 to 8 and 10, wherein the antigenic epitope is TAT-PTD sequence of human immune deficiency syndrome virus (HIV) (Tyr-Gly-Arg-Lys-Lys-Arg-Arg-Gln-Arg-Arg-Arg) (SEQ ID NO: 2) or amino acid sequence 3xRGD comprising integrin recognition sequence (RGD) (Arg-Gly-Asp-Arg-Gly-Asp-Arg-Gly-Asp) (SEQ ID NO: 3).

12. A virus-like particle formed from the altered capsid protein of SV40 set forth in claims 4 to 11.

13. The virus-like particle according to claim 12, wherein a biologically active substance is encapsulated therein.

14. The virus-like particle according to claim 13, wherein the biologically active substance is a pharmaceutically active ingredient.

15. The virus-like particle according to claim 13, wherein the biologically active substance is a nucleic acid for gene therapy.

16. A pharmaceutical composition comprising the virus particle set forth in claim 14.
